# FASCICULE DE BREVET EUROPEEN

(11) **EP 2 100 572 B1**
(45) Date de publication et mention de la délivrance du brevet: **29.09.2010**
(21) Numéro de dépôt: 08152611.3
(22) Date de dépôt: 11.03.2008
(51) Int. Cl.: A61C 8/00, A61B 17/88, B25B 23/142, B25B 13/46

(54) **Outil dynamométrique à tête amovible**
Dynamometrisches Instrument mit abnehmbarem Kopf
Torque tool with removable head

(43) Date de publication de la demande: 16.09.2009
(73) Titulaire: Hader SA, 2300 La Chaux-de-Fonds (CH)
(72) Inventeur: Miletto, Philippe, 1317 Orny (CH)
(74) Mandataire: GLN

(56) Documents cités:
- EP-A- 0 704 281
- EP-A- 1 834 734
- DE-U1-202004 014 195
- DE-U1-202007 015 689

## Description

### Domaine technique

La présente invention se rapporte au domaine de l'outillage médical et concerne un outil dynamométrique d'axe longitudinal AA, apte à fournir un couple de serrage comprenant :
- une tête munie d'un premier logement disposé selon l'axe longitudinal et un deuxième logement circulaire disposé selon un axe transversal, perpendiculaire à l'axe longitudinal, la tête étant destinée à recevoir une douille dans le deuxième logement,
- un manche destiné à prendre place dans le premier logement, le manche étant susceptible d'occuper une position de travail dans laquelle une première de ses extrémités est située dans le deuxième logement pour coopérer avec la douille,

Un tel outil est particulièrement utilisé en chirurgie sous la forme de clé, appelée usuellement clé sextant, notamment dans le domaine dentaire pour la pose d'implants.

### Etat de la technique

Les clés sextants sont bien connues de l'homme du métier et sont largement utilisées pour l'application de couples de serrage. Certains modèles sont dotés de limitateurs de couple et/ou d'indicateurs destinés à donner une valeur numérique à l'utilisateur lors de l'étape de serrage.

De manière générale, ce type de clé est constitué d'une tête où viennent se loger une douille de serrage ainsi qu'un manche, ce dernier travaillant avec la douille lors de l'étape de serrage. Ces clés peuvent comprendre un ressort qui agit sur la position de l'extrémité du manche lors de la rotation de la clé dans le sens inverse au serrage.

L'inconvénient principal des systèmes connus est que, une fois mis en place, le manche n'est que partiellement ou pas du tout démontable de la tête du dispositif, rendant compliqué de ce fait le nettoyage de l'ensemble, qui est particulièrement important dans le domaine médical.

Par ailleurs, la douille de serrage doit pouvoir être changée facilement. Cependant, dans les outils de l'état de la technique, elle n'est pas bloquée selon l'axe transversal de manière satisfaisante lorsque le manche est engagé dans la tête et peut donc être amené à tomber du dispositif en cours d'utilisation. Le document EP1834734 divulgue un outil dynanométrique selon le préambule de la revendication 1. d'utilisation.

La présente invention propose de fournir un outil dynamométrique exempt des inconvénients ci-dessus. Elle trouve une application particulièrement avantageuse sous la forme d'une clé sextant.

### Divulgation de l'invention

Plus particulièrement, l'invention porte sur un outil dynamométrique tel que mentionné ci-dessus, dans lequel la tête et la première extrémité du manche sont agencées et dimensionnées de manière à ce que la tête et le manche puissent occuper une première orientation relative dans laquelle le manche peut coulisser libremant par rapport à la tête de sorte que la tête est amovible du manche, et une deuxième orientation relative dans laquelle des éléments d'appui sont agencés de manière à maintenir le manche en position de travail.

### Brève description des dessins

D'autres caractéristiques de la présente invention apparaîtront plus clairement à la lecture de la description qui va suivre, faite en référence aux dessins annexés, dans lesquels:
- la figure 1 montre une vue en détail de l'agencement de l'extrémité de la clé sextant une fois montée et une vue en coupe supplémentaire selon l'axe indiqué,
- les figures 2, 3, 4 et 5 montrent diverses étapes d'assemblage d'un outil selon l'invention, avec des vues indicées a, en coupe selon l'axe longitudinale AA, et des vues en coupe indicées b, selon les axes indiqués.

### Mode(s) de réalisation de l'invention

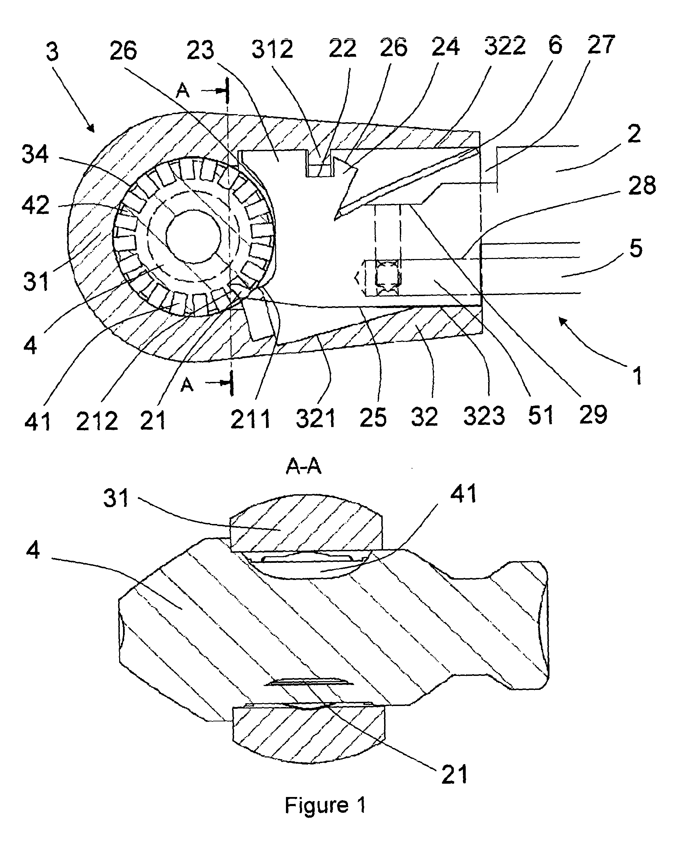

La figure 1 présente une clé sextant 1 selon l'invention, prête à être utilisée. La clé définit un axe longitudinal AA et un axe transversal BB, représentés sur les dessins.

La clé 1 comporte un manche 2 ayant une forme générale de barreau. Il présente une surface inférieure 25 et une surface supérieure 29 parallèles à l'axe AA. Le manche définit également une première extrémité constituée d'une paroi concave 26 dont la direction générale est sensiblement perpendiculaire à la surface supérieure 29, terminée à sa base par un bec 21 prolongeant la surface inférieure 25. Le bec 21 comporte un flanc incurvé 211 et un flanc sensiblement droit 212. Le flanc droit 212 est situé dans le prolongement de la surface inférieure 25 du manche 2.

La surface supérieure 29 du manche 2 est dotée d'une gorge 22 définissant un premier rebord 23 et un deuxième rebord 24. Le deuxième rebord 24 est situé du coté opposé à la première extrémité et présente une échancrure 26 à son extrémité, dont le rôle apparaitra ci-après. La surface supérieure 29 est également dotée d'une cuvette 27 destinée à accueillir un ressort 6, dont l'utilité sera également expliquée plus loin. Le manche 2 comporte encore une cavité allongée 28 placée dans sa partie inférieure et d'axe parallèle à la surface 25.

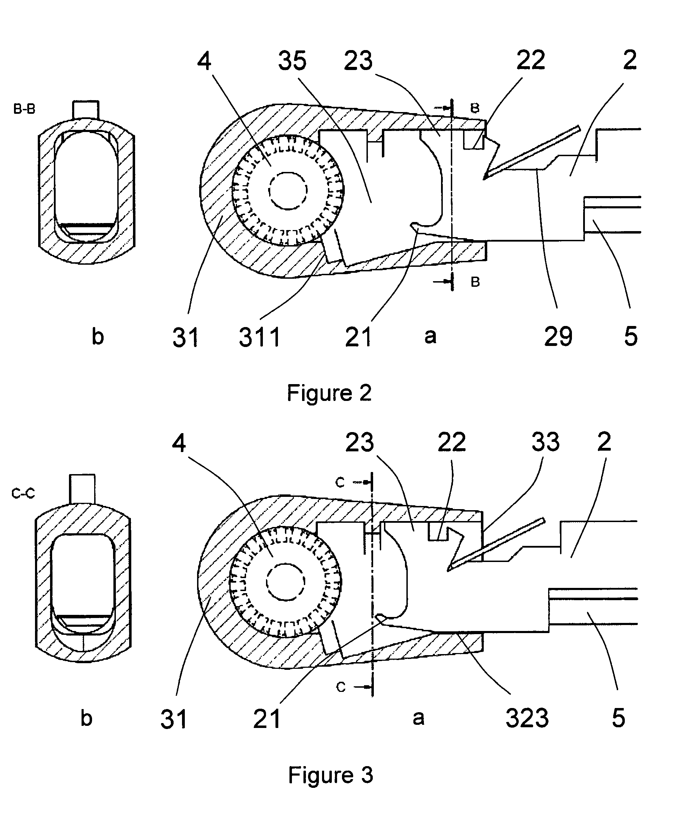

La clé 1 comporte également une tête 3 comprenant une extrémité semi-circulaire 31 prolongée par une partie de forme trapézoïdale 32. L'intérieur de la tête 3 est formé d'un premier logement 35 (voir figure 2), d'axe longitudinal AA, se situe au niveau de la partie trapézoïdale 32 de la tête 3 et débouche à l'extérieur, formant ainsi une ouverture 33 (voir figure 3). Le logement 35 comporte une paroi inférieure 321, terminée par une surface sensiblement perpendiculaire 311 (figure 2) dont le rôle sera évoqué plus loin, et une paroi supérieure 322. La paroi supérieure 322 est parallèle à l'axe longitudinal AA. Elle est dotée d'un ergot 312, typiquement situé aux deux tiers de sa longueur depuis l'ouverture 33. La paroi inférieure 321 de la cavité 35 est inclinée par rapport à l'axe longitudinal AA de telle sorte que les deux parois 321 et 322 divergent vers l'intérieur de la cavité 35.

L'intérieur de la tête 3 est également formé d'un deuxième logement 34, cylindrique d'axe BB, situé au niveau de l'extrémité 31 de la tête 3. Il se trouve en jonction avec le premier logement 35. De préférence, le logement 34 est concentrique avec la zone 31.

La clé 1 comporte également une douille de serrage 4, dotée à sa surface de cannelures 41 séparées entre elles par des parties 42. Elle est insérée dans le logement 34 de la tête 3 et disposée selon l'axe BB. Lorsque la douille est positionnée dans le logement 34, les cannelures sont orientées selon l'axe BB également. Comme le montre la coupe A-A de la figure 1, les cannelures 41 n'ont pas été usinées sur toute la hauteur de la douille 4 mais simplement sur une portion ne débouchant sur aucune des deux extrémités de la douille 4. Les cannelures 41 possèdent ainsi une longueur inférieure à la hauteur totale de la douille de serrage 4. La douille 4 est placée de manière à ce qu'une partie des cannelures 41 soit située en regard de l'extrémité du logement 35.

La première extrémité du manche 2 et l'ouverture 33 sont dimensionnées de manière à ce que la tête 3 soit librement amovible du manche 2, tout en définissant une position de travail sécurisée, dans laquelle la tête et le manche sont solidaires en translation. Plus particulièrement, il est prévu d'introduire le manche 2 dans la tête 3 en disposant ces deux éléments selon une première orientation relative. Ainsi, l'introduction se fait en plaçant la surface inférieure 25 dudit manche 2 en contact avec la paroi inférieure 321 du logement 35, parallèlement à cette dernière. La hauteur de l'extrémité du manche 2 entre sa surface inférieure 25 et la surface supérieure du rebord 23 est inférieure à la hauteur définie par les surfaces en regard 321 et 322, perpendiculairement à la surface 321. Ainsi, lorsque le manche est orienté en référence à la tête, parallèlement à la surfaces 321, il est possible de le faire translater jusqu'à que le bec 21 vienne en butée contre la surface 311.

L'ergot 312 et la gorge 22 sont positionnés et dimensionnés de manière à ce que, lorsque le bec 21 est en butée contre la surface 311, le rebord 23 ait franchi l'ergot 312. En outre et de manière avantageuse, l'ergot 312 et la gorge 22 sont agencés pour permettre que, lorsque le bec 21 est en butée contre la surface 311, le manche 2 peut être pivoté de telle sorte que l'ergot 312 vienne s'engager dans la gorge 22. L'échancrure 26 du rebord 24 est particulièrement conformée de manière à permettre ce pivotement, sans que le rebord 24 n'entre en contact contre la paroi 322 de la tête 3. Les rapports dimensionnels entre l'ergot et la gorge permettent de bloquer le manche 2 en translation suivant l'axe longitudinal AA, vers l'avant et vers l'arrière. La surface 25 dudit manche 2 n'est en contact avec la paroi 321 que par une surface d'appui 323 à l'extrémité débouchante de la paroi 321. Cette surface d'appui 323, d'une part, l'ergot 312 et la gorge 22, d'autre part, forment des éléments d'appuis qui permettent de définir la position de travail du manche, dans laquelle le bec 21 est susceptible de coopérer avec les cannelures 41 de la douille 4.

Ainsi, compte tenu de la structure des cannelures 41 décrite ci-dessus, la douille de serrage 4 est bloquée en translation selon l'axe BB, tant que le manche 2 est dans sa position de travail.

On comprend que, pour le désassemblage de la clé sextant 1, c'est l'opération inverse qui est effectuée en inclinant le manche 2 jusqu'à ce que la surface 25 soit parallèle à et en contact avec la paroi intérieure 321 de la cavité 35. Il suffira alors de faire translater le manche 2 le long de cette paroi pour terminer son extraction et complètement dissocier la tête et le manche.

Le ressort 6 placé dans la cuvette 27, est destiné à venir en contact avec la paroi 322 du logement 35. Ce ressort 6 intervient lorsque la clé 1 est mise en rotation dans le sens opposé au serrage (sens antihoraire) et exerce une pression sur le manche 2 qui permet le retour du bec 21, présent à l'extrémité dudit manche 2, dans la cannelure suivante de la douille de serrage 4, une fois la partie 42 séparant deux cannelures successives franchie.

La clé 1 comporte également une baguette flexible 5 dont l'une des extrémités est insérée dans la cavité 28 du manche 2. L'autre extrémité de ladite baguette flexible 5 est libre et peut évoluer en regard à une échelle graduée, permettant d'indiquer le couple exercé. L'utilisateur peut donc agir sur la baguette 5 lorsqu'il veut exercer un couple contrôlé et il agira directement sur le manche 2 lorsqu'il veut exercer un couple maximal.

La baguette 5 est rendue solidaire du manche 2 au moyen d'une goupille non représentée sur le dessin. La goupille est agencée de manière à maintenir en position la baguette flexible 5 en coopérant avec une rainure annulaire que comporte la baguette 5. Ainsi, on évite toute modification des caractéristiques mécaniques de la baguette 5, car celle-ci ne subit aucun traitement contraignant ni thermique ni mécanique.

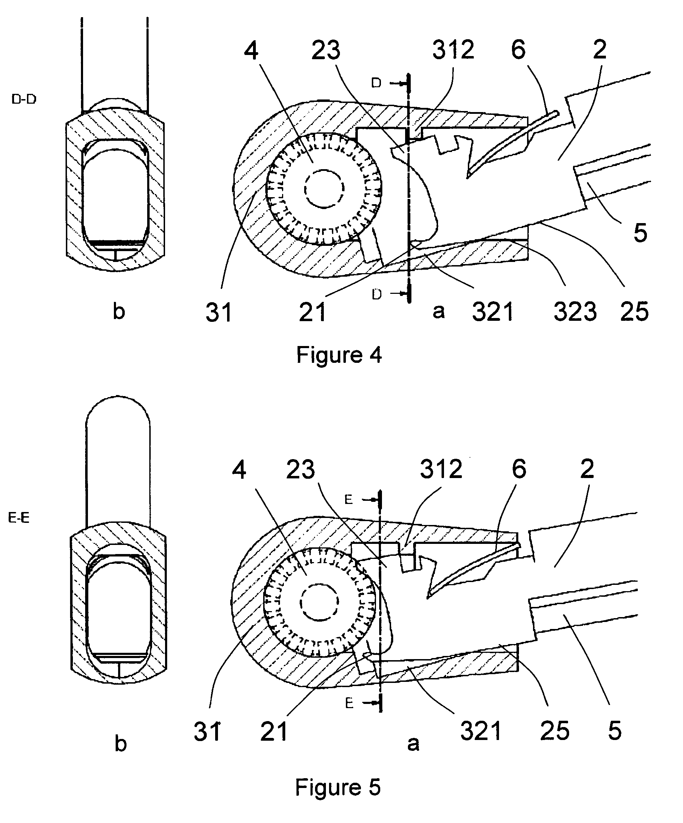

Les figures 2, 3, 4 et 5 montrent un procédé d'assemblage de la clé sextant 1, avec des vues 2a, 3a, 4a et 5a selon l'axe longitudinale AA et des vues en coupe 2b, 3b, 4b et 5b selon les axes indiqués. Plus particulièrement, elles représentent le procédé d'assemblage de la tête 3 et du manche 2 de la clé sextant 1 décrite ci-dessus. Sur ces figures, la douille 4 est partiellement représentée en traits discontinus car elle n'est normalement placée qu'après l'étape correspondant à la figure 5.

On dispose tout d'abord le manche 2 et la tête 3 dans leur première position relative. Puis la première extrémité du manche 2 est introduite dans le premier logement 35 via l'ouverture 33 et est translatée à l'intérieur du logement 35 en conservant la première orientation relative du manche 2 et de la tête 3. Lorsque que la translation est terminée, c'est-à-dire que la première extrémité 21 est en butée contre la surface 311, le manche 2 et la tête 3 sont disposés dans leur deuxième position relative, de manière à ce que le manche 2 soit placé dans sa position de travail. De manière avantageuse, lorsque le manche 2 est basculé pour que ce dernier et la tête 3 soient mis dans leur deuxième oritentation, ils sont directement positionnés pour permettre que les éléments d'appuis maintiennent la position de travail. Plus particulièrement lorsque le manche est mis en butée contre la surface 311 puis que le manche et la tête sont mis dans leur deuxième orientation relative, l'ergot 312 est introduit dans la gorge 22 par ces opérations.

Par la suite, l'extraction du manche 2, pour un entretien ou une stérilisation, se fera en suivant les étapes précédentes dans le déroulement inverse.

Ainsi est obtenu un outil dynamométrique dont la tête est très facilement et simplement amovible par l'avant, c'est-à-dire du côté de la première extrémité du manche, tout en garantissant un parfait maintien de la postion de travail du manche en référence à la tête et en sécurisant la mise en place de la douille dans l'outil, en cours d'utilisation.

Bien que la description ci-dessus ait été faite en référence à une clé sextant, elle peut être appliquée à d'autres outils dynamométriques. L'exemple ci-dessus n'est pas limitatif et l'homme du métier pourra envisager d'autres modes de réalisation à sa portée, sans toutefois sortir du cadre de la présente invention.

## Revendications

1. Outil dynamométrique d'axe longitudinal AA, apte à fournir un couple de serrage comprenant:
- une tête (3) munie d'un premier logement (35) disposé selon l'axe longitudinal AA et un deuxième logement (34) circulaire disposé selon un axe transversal BB, perpendiculaire à l'axe longitudinal AA, ladite tête (3) étant destinée à recevoir une douille (4) dans ledit deuxième logement (34),
- un manche (2) destiné à prendre place dans le premier logement (35), ledit manche (2) étant susceptible d'occuper une position de travail dans laquelle une première de ses extrémités est située dans ledit deuxième logement (34) pour coopérer avec ladite douille (4),
**caractérisé en ce que** la tête (3) et la première extrémité du manche (2) sont agencés et dimensionnés de manière à ce que la tête (3) et le manche (2) puissent occuper une première orientation relative dans laquelle le manche (2) peut coulisser librement par rapport à la tête (3) de sorte que la tête (3) est amovible du manche (2) du côté de la première extrémité, et une deuxième orientation relative dans laquelle des éléments d'appui sont agencés de manière à maintenir le manche (2) en position de travail.

2. Outil selon la revendication 1, **caractérisé en ce que** le premier logement (35) définit une première paroi (322) et une deuxième paroi (321), la première paroi (322) étant parallèle audit axe longitudinal AA et la deuxième paroi (321) étant essentiellement orientée selon l'axe longitudinal AA sans y être parallèle,
et **en ce que** la hauteur entre les parois en référence à la deuxième paroi (321), est supérieure à la hauteur de ladite première extrémité dudit manche (2).

3. Outil selon l'une des revendications 1 et 2, **caractérisé en ce que** le logement (35) comporte un organe de butée agencé de manière à définir une position extrême du manche (2) en référence à la tête (3), lorsque le manche (2) et la tête (3) sont dans leur première orientation relative.

4. Outil selon l'une des revendications 2 et 3, **caractérisé en ce que** les éléments d'appuis comportent un ergot (312) situé sur la première paroi (322), et une gorge (22) dont est doté le manche (2), définie par un premier rebord (23) et un deuxième rebord (24), l'ergot (312) et la gorge (22) étant agencés de manière à ce que l'ergot (312) soit positionné dans la gorge (22) lorsque le manche (2) est en position de travail.

5. Outil selon la revendication précédente, **caractérisé en ce que** l'organe de butée, la gorge (22) et l'ergot (312) sont agencés de manière à ce que, lorsque le manche (2) est dans ladite position extrême, le passage du manche (2) et de la tête (3) de leur première à leur deuxième orientations relatives permet l'engagement de l'ergot (312) dans la gorge (22).

6. Outil selon l'une des revendications 4 et 5, **caractérisé en ce que** les éléments d'appuis comportent en outre l'extrémité (323) de la deuxième paroi (321).

7. Outil selon l'une des revendications précédentes, **caractérisé en ce qu'**il comporte en outre une baguette flexible (5) dont l'une des extrémités (51) est solidaire du manche (2).

8. Outil selon la revendication 7, **caractérisé en ce que** ladite baguette (5) est montée fol dans une ouverture (28) que comporte le manche (2), un élément de blocage solidarisant le manche (2) et la baguette (5).

9. Outil selon la revendication 8, **caractérisé en ce que** l'élément de blocage est une goupille, la baguette (5) comportant une rainure annulaire avec laquelle coopère la goupille.

10. Outil selon l'une des revendications précédentes, comportant une douille (4) disposée dans le deuxième logement (34), ladite douille (4) étant munie de cannelures (41) à sa surface, l'extrémité du manche (2) étant munie d'un bec (21) destiné à s'engager dans une cannelure (41) de la douille (4) pour entraîner ladite douille (4) en rotation, **caractérisé en ce que** les extrémités des cannelures (41) de la douille de serrage (4) ne sont pas débouchantes selon l'axe transversal, le bec (21) du manche (2) étant susceptible de bloquer en translation verticale la douille (4).

11. Procédé d'assemblage d'une tête (3) et d'un manche (2) d'un outil dynamométrique selon l'une des revendications précédentes, comprenant les étapes suivantes :
- disposition du manche (2) et de la tête (3) dans leur première orientation relative,
- introduction de la première extrémité du manche (2) dans ledit premier logement (35) et translation du manche (2) à l'intérieur dudit premier logement (35), tout en conservant la première orientation relative du manche (2) et de la tête (3),
- disposition du manche (2) et de la tête (3) dans leur deuxième orientation relative de manière à mettre le manche (2) dans sa position de travail et ladite position de travail étant maintenue par les éléments d'appui.

12. Procédé d'assemblage selon la revendication 11 d'un outil selon l'une des revendications 3 à 10, **caractérisé en ce que** l'étape d'introduction se fait jusqu'à mettre le manche (2) en butée contre l'organe de butée (311) de la tête (3).

13. Procédé d'assemblage selon la revendication 12 d'un outil selon l'une des revendications 4 à 10, **caractérisé en ce que** lorsque le manche (2) et la tête (3) sont disposés dans leur deuxième orientation, la position de travail est directement maintenue par la coopération de l'ergot (312) et de la gorge (22).

## Claims

1. Dynamometric tool of longitudinal axis AA, capable of supplying a tightening torque, comprising:
- a head (3) provided with a first housing (35) arranged along the longitudinal axis AA and a circular second housing (34) arranged along a transverse axis BB, perpendicular to the longitudinal axis AA, said head (3) being designed to receive a bushing (4) in said second housing (34),
- a sleeve (2) designed to be placed in the first housing (35), said sleeve (2) being able to occupy a working position in which a first of its ends is located in said second housing (34) so as to cooperate with said bushing (4),
**characterised in that** the head (3) and the first end of the sleeve (2) are arranged and dimensioned in such a way that the head (3) and the sleeve (2) can occupy a first relative orientation in which the sleeve (2) can slide freely with respect to the head (3) so that the head (3) is removable from the sleeve (2) on the side of the first end, and a second relative orientation in which bearing elements are arranged so as to maintain the sleeve (2) in the working position.

2. Tool according to claim 1, **characterised in that** the first housing (35) defines a first wall (322) and a second wall (321), the first wall (322) being parallel to said longitudinal axis AA and the second wall (321) being essentially oriented along the longitudinal axis AA without being parallel thereto,
and **in that** the height between the walls with reference to the second wall (321) is greater than the height of said first end of said sleeve (2).

3. Tool according to one of claims 1 and 2, **characterised in that** the housing (35) comprises a stop member arranged so as to define an end position of the sleeve (2) with reference to the head (3), when the sleeve (2) and the head (3) are in their first relative orientation.

4. Tool according to one of claims 2 and 3, **characterised in that** the bearing elements comprise a lug (312) located on the first wall (322) and a notch (22) provided on the sleeve (2) and defined by a first edge (23) and a second edge (24), the lug (312) and the notch (22) being arranged so that the lug (312) is positioned in the notch (22) when the sleeve (2) is in the working position.

5. Tool according to the preceding claim, **characterised in that** the stop member, the notch (22) and the lug (312) are arranged so that, when the sleeve (2) is in said end position, the passage of the sleeve (2) and of the head (3) from their first to their second relative orientation allows the lug (312) to engage in the notch (22).

6. Tool according to one of claims 4 and 5, **characterised in that** the bearing elements additionally comprise the end (323) of the second wall (321).

7. Tool according to one of the preceding claims, **characterised in that** it additionally comprises a flexible rod (5), one of the ends (51) of which is secured to the sleeve (2).

8. Tool according to claim 7, **characterised in that** said rod (5) is mounted idle in an opening (28) on the sleeve (2), a locking element securing the sleeve (2) and the rod (5).

9. Tool according to claim 8, **characterised in that** the locking element is a pin, the rod (5) comprising an annular groove with which the pin cooperates.

10. Tool according to one of the preceding claims, comprising a bushing (4) arranged in the second housing (34), said bushing (4) being provided with grooves (41) on its surface, the end of the sleeve (2) being provided with a protrusion (21) which is designed to engage in a groove (41) of the bushing (4) so as to drive said bushing (4) in rotation, **characterised in that** the ends of the grooves (41) of the tightening bushing (4) are not open along the transverse axis, the protrusion (21) of the sleeve (2) being able to lock the bushing (4) in terms of vertical translation.

11. Method for assembling a head (3) and a sleeve (2) of a dynamometric tool according to one of the preceding claims, comprising the following steps:
- arranging the sleeve (2) and the head (3) in their first relative orientation,
- introducing the first end of the sleeve (2) into said first housing (35) and moving the sleeve (2) in translation inside said first housing (35), while preserving the first relative orientation of the sleeve (2) and of the head (3),
- arranging the sleeve (2) and the head (3) in their second relative orientation so as to place the sleeve (2) in its working position, and said working position being maintained by the bearing elements.

12. Assembly method according to claim 11 for a tool according to one of claims 3 to 10, **characterised in that** the introduction step takes place until the sleeve (2) is in abutment against the stop member (311) of the head (3).

13. Assembly method according to claim 12 for a tool according to one of claims 4 to 10, **characterised in that**, when the sleeve (2) and the head (3) are arranged in their second orientation, the working position is directly maintained by the cooperation of the lug (312) and the notch (22).

## Patentansprüche

1. Dynamometrisches Werkzeug mit einer Längsachse AA, dazu geeignet, ein Anzugsmoment zu liefern, umfassend:
- einen Kopf (3), ausgestattet mit einem ersten Aufnahme (35), die gemäß der Längsachse AA angeordnet ist, und eine zweite runde Aufnahme (34), die gemäß der Querachse BB angeordnet ist, senkrecht zur Längsachse AA, wobei der Kopf (3) dazu vorgesehen ist, in der zweiten Aufnahme (34) eine Buchse (4) aufzunehmen,
- einen Griff (2), der dazu vorgesehen ist, in der ersten Aufnahme (35) angeordnet zu sein, wobei der Griff (2) dazu geeignet ist, eine Arbeitsposition einzunehmen, in der ein erstes seiner Enden in der zweiten Aufnahme (34) angeordnet ist, um mit der Buchse (4) zusammen zu arbeiten,
**dadurch gekennzeichnet, dass** der Kopf (3) und das erste Ende des Griffs (2) so angeordnet und dimensioniert sind, dass der Kopf (3) und der Griff (2) eine erste relative Ausrichtung einnehmen können, in der der Griff (2) in Bezug auf den Kopf (3) frei gleiten kann, so dass der Kopf (3) von der Seite des ersten Endes vom Griff (2) entfernt werden kann, und eine zweite Ausrichtung bezüglich der Stützelemente angeordnet sind, um den Griff (2) in der Arbeitsposition zu halten.

2. Werkzeug nach Anspruch 1, **dadurch gekennzeichnet, dass** die erste Aufnahme (35) eine erste Wand (322) und eine zweite Wand (321) definiert, wobei die erste Wand (322) parallel zur Längsachse AA ist und die zweite Wand (321) im Wesentlichen gemäß der Längsachse AA ausgerichtet ist, ohne parallel dazu zu sein, und **dadurch**, dass die Höhe zwischen den Wänden bezüglich der zweiten Wand (321) größer als die Höhe des ersten Endes des Griffs (2) ist.

3. Werkzeug nach einem der Ansprüche 1 und 2, **dadurch gekennzeichnet, dass** die Aufnahme (35) ein Anschlagorgan umfasst, das derart angebracht ist, dass es eine Endposition des Griffs (2) bezüglich des Kopfs (3) definiert, wenn sich der Griff (2) und der Kopf (3) in ihrer ersten relativen Ausrichtung befinden.

4. Werkzeug nach einem der Ansprüche 2 und 3, **dadurch gekennzeichnet, dass** die Stützelemente einen Vorsprung (312) aufweisen, der sich auf der ersten Wand (322) befindet, und eine Auskehlung (22), mit der der Griff (2) versehen ist, definiert durch einen ersten Rand (23) und einen zweiten Rand (24), wobei der Vorsprung (312) und die Au8skehlung (22) so angebracht sind, dass der Vorsprung (312) in der Auskehlung (22) positioniert ist, wenn sich der Griff (2) in der Arbeitsposition befindet.

5. Werkzeug nach dem vorhergehenden Anspruch, **dadurch gekennzeichnet, dass** das Anschlagorgan, die Auskehlung (22) und der Vorsprung (312) so angebracht sind, dass, wenn sich der Griff (2) in der Endposition befindet, der Übergang des Griffs (2) und des Kopfs (3) von ihrer ersten in ihre zweite relative Ausrichtung ein Eingreifen des Vorsprungs (312) in die Auskehlung (22) ermöglicht.

6. Werkzeug nach einem der Ansprüche 4 und 5, **dadurch gekennzeichnet, dass** die Stützelemente außerdem das Ende (323) der zweiten Wand (321) umfassen.

7. Werkzeug nach einem der vorhergehenden Ansprüche, **dadurch gekennzeichnet, dass** es außerdem eine flexible Leiste (5) umfasst, deren eines Ende (51) fest mit dem Griff (2) verbunden ist.

8. Werkzeug nach Anspruch 7, **dadurch gekennzeichnet, dass** die Leiste (5) lose in einer Öffnung (28) angebracht ist, die den Griff (2) umfasst, wobei ein Sperrelement, das den Griff (2) und die Leiste (5) fest miteinander verbindet.

9. Werkzeug nach Anspruch 8, **dadurch gekennzeichnet, dass** das Sperrelement ein Stift ist, wobei die Leiste (5) eine ringförmige Nut umfasst, die mit dem Stift zusammenarbeitet.

10. Werkzeug nach einem der vorhergehenden Ansprüche, umfassend eine Buchse (4), die in der zweiten Aufnahme (34) angebracht ist, wobei die Buchse (4) mit Rillen (41) an ihrer Oberfläche versehen ist, wobei das Ende des Griffs (2) mit einem Schnabel (21) versehen ist, der dazu vorgesehen ist, in eine Rille (41) der sich drehenden Buchse (4) einzugreifen, **dadurch gekennzeichnet, dass** die Enden der Rillen (41) der Spannbuchse nicht entlang der Querachse münden, wobei der Schnabel (21) des Griffs (2) dazu geeignet ist, die Buchse (4) in vertikaler Drehung zu blockieren.

11. Verfahren zur Montage eines Kopfes (3) und eines Griffs (2) eines dynamometrischen Werkzeugs nach einem der vorgehenden Ansprüche, umfassend die folgenden Schritte:
- Anbringen des Griffs (2) und des Kopfes (3) in ihrer ersten relativen Ausrichtung,
- Einführen des ersten Endes des Griffs (2) in die erste Aufnahme (35) und Übertragen des Griffs (2) in das Innere der ersten Aufnahme (35), während gleichzeitig die erste relative Ausrichtung des Griffs (2) und des Kopfes (3) beibehalten wird.
- Anbringen des Griffs (2) und des Kopfs (3) in ihrer zweiten relativen Ausrichtung, um den Griff (2) in seine Arbeitsposition zu bringen, und wobei die Arbeitsposition von den Stützelemente aufrecht erhalten wird.

12. Montageverfahren nach Anspruch 11 eines Werkzeugs nach einem der Ansprüche 3 bis 10, **dadurch gekennzeichnet, dass** der Schritt des Einführens erfolgt, bis der Griff (2) im Anschlag gegen das Anschlagorgan (311) des Kopfes (3) eingeführt ist.

13. Montageverfahren nach Anspruch 12 eines Werkzeugs nach einem der Ansprüche 4 bis 10, **dadurch gekennzeichnet, dass**, wenn der Griff (2) und der Kopf (3) in ihrer zweiten Ausrichtung angeordnet sind, die Arbeitsposition direkt von der Zusammenarbeit des Vorsprungs (312) und der Auskehlung (22) aufrecht erhalten wird.
